# EUROPEAN PATENT SPECIFICATION

(11) **EP 3 064 144 B1**
(45) Date of publication and mention of the grant of the patent: **10.10.2018**
(21) Application number: 16158650.8
(22) Date of filing: 04.03.2016
(51) Int. Cl.: A61B 17/072, B29C 65/08

(54) **JAW MEMBERS AND METHODS OF MANUFACTURE**
BACKENELEMENTE UND VERFAHREN ZUR HERSTELLUNG
ÉLÉMENTS DE MÂCHOIRE ET PROCÉDÉS DE FABRICATION

(30) Priority: 05.03.2015 US 201514639338
(43) Date of publication of application: 07.09.2016
(73) Proprietor: Covidien LP, Mansfield, MA 02048 (US)
(72) Inventor: NUKALA, Rajasekhar, 500011 Secunderabad (IN); PINJALA, Venkata Ramana Mohan, Eendalnagar (IN)
(74) Representative: Soames, Candida Jane

(56) References cited:
- CN-A- 102 885 641
- US-A1- 2010 094 315
- US-A1- 2014 367 447
- US-B1- 8 087 562
- Sophie Morneau: "Wayback machine date stamp for: Ultrasonic Plastic Joining", , 18 February 2015 (2015-02-18), XP055311300, Retrieved from the Internet: URL:web.archive.org/web/*/http://www.emers onindustrial.com/en-US/documentcenter/Bran sonUltrasonics/Plastic%20Joining/Ultrasoni cs/Technical%20Info/PW-3_Part_Design_for_U ltrasonic_Welding_%28single_pgs%29_hr.pdf [retrieved on 2016-10-17] & Sophie Morneau: "Ultrasonic Plastic Joining Technical Information Part Design for Ultrasonic Welding Primary Factors Influencing Joint Design", , 18 February 2015 (2015-02-18), XP055311308, Retrieved from the Internet: URL:www.emersonindustrial.com/en-US/docume ntcenter/BransonUltrasonics/Plastic Joining/Ultrasonics/Technical Info/PW-3_Part_Design_for_Ultrasonic_Weldi ng_(single_pgs)_hr.pdf [retrieved on 2016-10-17]

## Description

### BACKGROUND

### Technical field

The present disclosure relates generally to surgical instruments and, more specifically, to surgical instruments for surgically joining tissue and methods of manufacturing/assembling jaw members of surgical instruments.

### Background of Related Art

Surgical stapling instruments used for applying parallel rows of staples through compressed living tissue are well known in the art. These surgical instruments are commonly employed for closing tissue or organs prior to transaction or resection, for occluding organs in thoracic and abdominal procedures, and for fastening tissue in anastomoses.

Typically, such surgical stapling instruments include an anvil assembly, a cartridge assembly for supporting an array of surgical staples, an approximation mechanism for approximating the anvil and cartridge assemblies, and a firing mechanism for ejecting the surgical staples from the cartridge assembly.

In use, a surgeon initially approximates the anvil and cartridge assemblies. Next, the surgeon can actuate the surgical instrument to place staples in tissue. Additionally, the surgeon may use the same surgical instrument or a separate instrument to cut the tissue adjacent or between the row(s) of staples.

Typically, to manufacture anvil and cartridge assemblies of these surgical stapling instruments, sheet metal processing, stamping, and machining, *inter alia*, and laser welding for assembly are utilized. These machining operations can be complex and costly. Accordingly, a need exists for a more cost effective and simplified process of manufacturing components of surgical stapling instruments.

### SUMMARY

In one embodiment of the present disclosure, a pre-assembly for producing an anvil assembly for use with a surgical stapling instrument is provided. The anvil assembly includes an anvil plate and an anvil cover each fabricated from a thermoplastic material. The anvil plate has a knife channel defined longitudinally therethrough. The anvil plate includes a first surface and a second surface opposite the first surface. The first surface has a plurality of beads disposed along a periphery thereof. The anvil cover is configured to be coupled to a distal end of the surgical stapling instrument. The anvil cover includes a surface having at least one elongate surface feature disposed along a periphery thereof. The lateral beads of the anvil plate and the elongate surface feature of the anvil cover are configured for abutting engagement upon assembly thereof.

In certain embodiments, the plurality of beads of the anvil plate are a plurality of lateral beads disposed along a periphery thereof. The at least one elongate surface feature can be at least one longitudinal bead feature.

In some embodiments, the beads may have a triangular configuration and the elongate surface feature may have a triangular transverse cross section configuration.

It is contemplated that the thermoplastic material may be polyetheretherketone.

It is envisioned that an end of the anvil plate and an end of the anvil cover may each define an opening therethrough such that upon assembly of the anvil plate with the anvil cover, the openings are in overlapping alignment.

In some embodiments, the anvil plate may further include a pair of posts extending perpendicularly from an end of the second surface thereof. The anvil plate may further include a pair of biasing members engaged to the pair of posts.

It is contemplated that the anvil plate may further include a wall extending perpendicularly from the periphery of the first surface of the anvil plate. In some embodiments, the anvil plate and the anvil cover may each have an arcuate configuration.

The anvil assembly formed from the pre-assembly of the invention may be used with an end effector for use with a surgical stapling instrument. The end effector includes an anvil assembly and a cartridge assembly. The anvil assembly includes an anvil plate and an anvil cover each fabricated from a thermoplastic material. The anvil plate has a knife channel defined longitudinally therethrough. The anvil plate includes a first surface and a second surface opposite the first surface. The first surface has a plurality of beads disposed along a periphery thereof. The anvil cover is configured to be coupled to a distal end of a surgical stapling instrument. The anvil cover includes a surface having at least one elongate surface feature disposed along a periphery thereof. The beads of the anvil plate and the elongated surface feature of the anvil cover are configured for abutting engagement upon assembly thereof. At least one of the anvil assembly or the cartridge assembly is movable relative to the other between a spaced position and an approximated
position to engage and staple tissue there between. The anvil assembly and the cartridge assembly may each have an arcuate configuration.

In yet another aspect of the present disclosure, a method of manufacturing an anvil assembly of a surgical stapling instrument is provided. The method includes providing an anvil plate and an anvil cover, and ultrasonically welding the anvil plate to the anvil cover at an interface between beads of the anvil plate and an elongate surface feature of the anvil cover.

In some embodiments, the method may further include aligning an opening defined in an end of the anvil plate with an opening defining in an end of the anvil cover prior to ultrasonically welding the anvil plate to the anvil cover.

### BRIEF DESCRIPTION OF FIGURES

Various embodiments of the presently disclosed surgical stapling instruments are disclosed herein with reference to the drawings, wherein:
FIG. 1 is a perspective view of a surgical stapling instrument including an end effector in accordance with an embodiment of the present disclosure;
FIG. 2A is a top, perspective view of an anvil plate of an anvil assembly of the end effector of FIG. 1;
FIG. 2B is a bottom, perspective view of the anvil plate of FIG. 2A;
FIG. 3A is a bottom, perspective view of an anvil cover of the anvil assembly of FIG. 1;
FIG. 3B is a top, perspective view of the anvil cover of FIG. 3A;
FIG. 4A is a bottom, perspective view of the anvil assembly of FIG. 1, in its assembled form; and
FIG. 4B is a top, perspective view of the anvil assembly of FIG. 4A.

### DETAILED DESCRIPTION

Embodiments of the presently disclosed surgical stapling instrument are described in detail with reference to the drawings, wherein like reference numerals designate similar or identical elements in each of the several views. In the drawings and the description that follows, the term "proximal" refers to the end of the surgical stapling instrument, or component thereof, that is closest to the operator, whereas the term "distal" refers to the end of the surgical stapling instrument, or component thereof, that is farthest from the operator. As appreciated by one skilled in the art, the depicted surgical stapling instrument fires staples, but it may be adapted to fire any other suitable fastener such as clips and two-part fasteners.

As used herein, the terms parallel and perpendicular are understood to include relative configurations that are substantially parallel and substantially perpendicular up to about +/- 10 degrees from true parallel and true perpendicular.

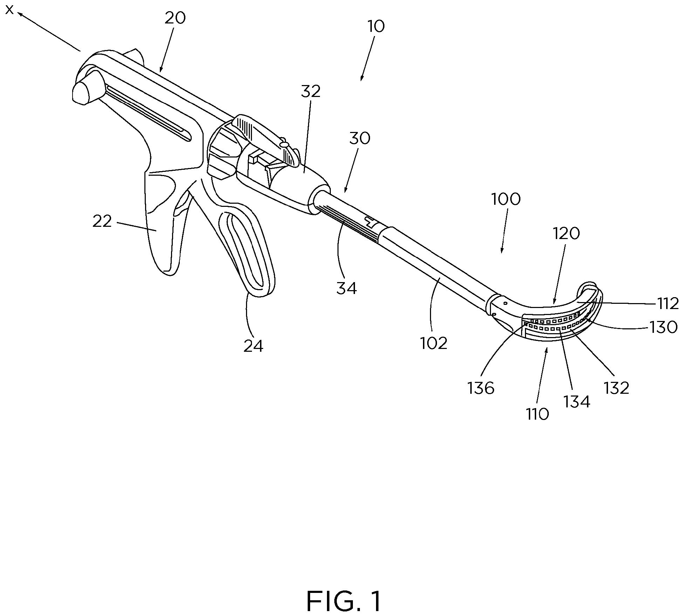

With reference to FIG. 1, reference numeral 10 designates an embodiment of the presently disclosed surgical stapling instrument. In the interest of brevity, the present disclosure focuses on jaw members 110, 120 of an end effector 100 of instrument 10 and, more specifically, an anvil assembly 112 of jaw member 120 of instrument 10. U.S. Patent Nos. 8,899,461 and 7,565,993 and U.S. Patent Application Serial No. 14/056,198, filed on October 17, 2013, describe in detail the structure and operation of other surgical end effectors.

Surgical stapling instrument 10 is configured to clamp, fasten, and/or cut tissue. In general, instrument 10 includes a handle assembly 20, an elongate portion or an adapter assembly 30 extending distally from handle assembly 20 and defining a longitudinal axis "X," and a curved end effector 100 adapted to clamp and fasten tissue. Adapter assembly 30 interconnects handle assembly 20 and end effector 100. Adapter assembly 30 includes a proximal housing 32 operatively coupled to a distal end of handle assembly 20 and a distal elongate portion 34 operatively coupled to a proximal elongate portion 102 of end effector 100. It is contemplated that the handle assembly can be a manually operated handle, such as that depicted in FIG. 1, a powered handle that is powered by a motor or some other driver, configured for attachment to a robotic system, etc.

Handle assembly 20 includes a stationary handle 22 and a movable handle 24. Movable handle 24 is adapted to move pivotally toward or away from stationary handle 22. Further, movable handle 24 is operatively coupled to end effector 100 through a mechanism adapted to convert at least a partial actuation of movable handle 24 into a pivoting motion of at least one of a first jaw member 110 or second jaw member 120 between spaced and approximated positions. As recognized by one skilled in the art, any conventional actuation mechanism may be employed to operatively couple movable handle 24 to end effector 100.

In disclosed embodiments, handle assembly 20 contains an actuation mechanism (not shown) for deploying fasteners, such as, for example, surgical staples from end effector 100 and advancing a knife (not shown) of end effector 100. The actuation mechanism includes a firing rod (not shown) operatively connected to movable handle 24. In operation, pivoting movable handle 24 toward stationary handle 22 causes the firing rod to advance distally. The firing rod is in turn operatively coupled to an axial drive assembly (not shown) at least partially positioned within end effector 100. The axial drive assembly is configured to move distally in response to a distal translation of the firing rod. Distal translation of the axial drive assembly causes first jaw member 110 to pivot toward second jaw member 120. In certain embodiments, the drive assembly has an elongate beam, with a distal head. The distal head has an upper portion for engaging one of the jaw members, and a lower portion for engaging the other jaw member. Alternatively, a tubular shaped member can be used to close the jaws. In addition, the axial drive assembly pushes an actuation sled (not shown) disposed within first jaw member 110, while the actuation sled translates through end effector 100. As the actuation sled advances through first jaw member 110, the actuation sled urges fasteners out of fastener retaining slots defined in first jaw member 110. In one embodiment, the axial drive assembly includes a blade (not shown) mounted on a distal portion thereof. In operation, the blade moves through end effector 100 when axial drive assembly moves distally through end effector 100 to cut tissue. As an alternative to the actuation sled, a series of cam bars can be advanced to deploy fasteners.

With continued reference to FIG. 1, end effector 100 includes proximal elongate portion 102, first jaw member 110, and second jaw member 120. In some embodiments, end effector 100 may be in the form of a disposable loading unit. In some embodiments, jaw members 110, 120 are each directly connected to a distal end of distal elongate portion 34 of adapter assembly 30. First and second jaw members 110, 120 have an arcuate shape (i.e., first and second jaw members 110, 120 are curved with respect to longitudinal axis "X"). It is envisioned that first and second jaw members 110, 120 may facilitate performing certain types of surgical procedures. For example, first and second jaw members 110, 120, as compared to straight jaw members, may help facilitate access to lower pelvic regions, e.g., during lower anterior resection ("LAR") or other colo-rectal surgery. First and second jaw members 110, 120 are movable, e.g., pivotable, relative to one another between a spaced-apart position and an approximated position to engage and staple tissue therebetween. Although shown in an arcuate configuration, it is contemplated that first jaw member 110 and second jaw member 120 can be linear.

First jaw member 110 includes a cartridge assembly 130 configured for retaining and discharging staples, and second jaw member 120 includes an anvil assembly 112 against which staples are formed during actuation of end effector 100. Cartridge assembly 130 has an arcuate configuration and includes a surface 132 configured to engage tissue. Surface 132 of cartridge assembly 130 generally faces anvil assembly 112 (see FIG. 1) and, during operation, engages tissue when anvil assembly 112 is approximated toward cartridge assembly 130.

Cartridge assembly 130 includes a plurality of fastener retaining slots 134 defined in surface 132 of cartridge assembly 130. Fastener retaining slots 134 are arranged in arcuate rows (e.g., two concentric rows) along surface 132 and extend along a curved length of cartridge assembly 130. Each fastener retaining slot 134 is adapted to hold a fastener (not shown) until a user actuates handle assembly 20. As mentioned above, when movable handle 24 is pivoted toward stationary handle 22, the fasteners are ejected from fastener retaining slots 134 and move toward anvil assembly 112.

Cartridge assembly 130 further includes a knife channel 136 defined in surface 132 and configured for translatable passage therethrough of a knife or other suitable cutting tool (not shown). Knife channel 136 is disposed between adjacent rows of fastener retainer slots 134 and extends along the curved length of cartridge assembly 130. In operation, a knife (not shown) slides through knife channel 136 when movable handle 24 pivots toward stationary handle 22. Alternately, other mechanisms can be used to drive the knife through knife channel 136. As mentioned above, actuating handle assembly 20 not only drives a knife along knife channel 136 to cut tissue disposed between anvil assembly 112 and cartridge assembly 130, but also ejects the fasteners from fastener retaining slots 134 (e.g., via a single stroke or multiple strokes of movable handle 24) into the tissue.

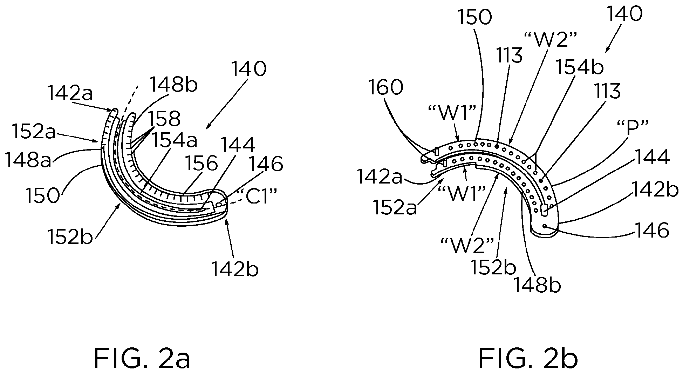

With reference to FIGS. 2A-4B , second jaw member 120 includes an anvil assembly 112 defining staple forming pockets 113 configured to deform staples ejected from cartridge assembly 130 upon approximation of jaw members 110, 120. The pockets are shaped to deform fasteners into a desired shape, such as the "B" shape of a typical staple. Other shapes and types of deformable fasteners are contemplated.

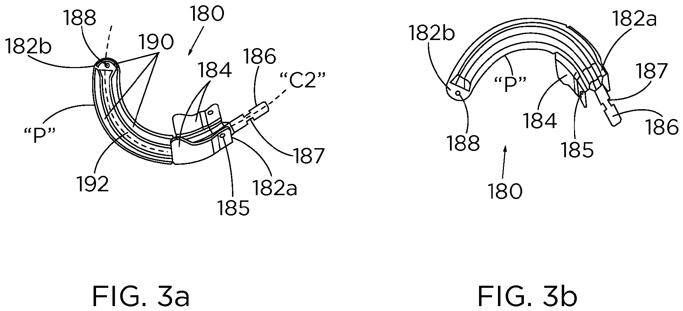

Anvil assembly 112 generally includes an anvil plate 140 (FIGS. 2A and 2B) and an anvil cover 180 (FIGS. 3A and 3B) coupled to one another and each being fabricated from a thermoplastic material. In some embodiments, components of, or an entirety of, anvil plate 140 and/or anvil cover 180 may be fabricated from various plastic materials, such as, for example, polyaryletherketone (PAEK) including polyetheretherketone (PEEK), polyetherketoneketone (PEKK) and polyetherketone (PEK), carbon-PEEK composites, polyethylene terephthalate (PET), silicone, polyurethane, silicone-polyurethane copolymers, semi-rigid and rigid materials. Anvil plate 140 and/or anvil cover 180 may have material composites, including the above materials, to achieve various desired characteristics such as strength, rigidity, elasticity, compliance, and durability. Anvil plate 140 and/or anvil cover 180 may also be fabricated from a heterogeneous material such as a combination of two or more of the above-described materials.

With reference to FIGS. 2A and 2B, anvil plate 140 has an arcuate configuration defining a curved central axis "C1" that extends between a first end 142a and a second end 142b of anvil plate 140. Anvil plate 140 has a knife channel 144 defined along curved central axis "Cl" through anvil plate 140. Second end 142b of anvil plate 140 has an opening 146 defined through a thickness thereof. Opening 146 is in alignment with curved central axis "Cl."

Anvil plate 140 has a first lateral side 148a and a second lateral side 148b that define a width of anvil plate 140 therebetween. The width of anvil plate 140 changes at a juncture 150 disposed between first and second ends 142a, 142b to define a first portion 152a of anvil plate 140 and a second portion 152b of anvil plate 140. First portion 152a of anvil plate 140 has a first width "Wl" (FIG. 2B) and second portion 152b has a second width "W2" (FIG. 2B) greater than first width "Wl" of first portion 152a such that the periphery "P" of second portion 152b is disposed a greater distance laterally from axis "Cl" than the periphery "P" of first portion 152a. In this way, the increased width of anvil plate 140 at second portion 152b provides a greater area for anvil plate 140 to accommodate beads 158 for ultrasonic welding, as described in greater detail below. It is contemplated that the width can be tapered, stepped, or some other transition can be provided.

Anvil plate 140 includes a first, top surface 154a having a planar configuration and a second, bottom surface 154b, opposite the first surface 154a, also having a planar configuration. In some embodiments, surfaces 154a, 154b may assume various configurations, such as, for example, undulating, tapered, or bent. First surface 154a of anvil plate 140 has a wall 156 extending perpendicularly therefrom. Wall 156 is disposed along periphery "P" of second portion 152b. In some embodiments, wall 156 may extend along periphery "P" of both first and second portions 152a, 152b of anvil plate 140. Wall 156 functions to guide the placement of anvil cover 180 into complementary alignment with anvil plate 140 during assembly, as will be described in greater detail below.

First surface 154a of anvil plate 140 further includes a plurality of lateral beads 158 disposed between periphery "P" of anvil plate 140 and axis "C1" of anvil plate 140. Beads 158 may have a triangular configuration. In some embodiments, beads 158 may assume a variety of shapes and configurations, such as, for example, square, round, oval, pyramidal, star-shaped, or the like. Beads 158 extend from first surface 154a of anvil plate 140 a distance that is less than a distance wall 156 extends from first surface 154a. Beads 158 that are supported on second portion 152b of anvil plate 140 are disposed further laterally from curved central axis "Cl" than are the beads 158 supported on first portion 152a of anvil plate 140. As such, and as can be appreciated, an imaginary line intersecting the beads 158 that are supported on first portion 152a, and an imaginary line intersecting the beads 158 that are supported on second portion 152b, are parallel but are not coaxial. In some embodiments, all of beads 158 may be in alignment with one another. It is contemplated that beads 158 may be fabricated from a variety of materials, such as, for example, those materials already described herein.

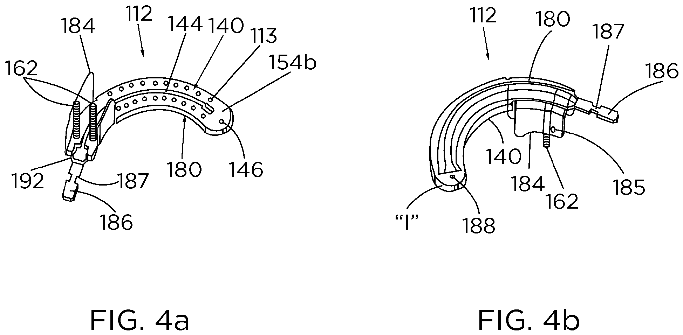

With reference to FIG. 2B, second surface 154b of anvil plate 140, which faces cartridge assembly 130 upon assembly of end effector 100, has a pair of posts 160 extending perpendicularly from first end 142a of anvil plate 140. Posts 160 are disposed in a side-by-side orientation such that an imaginary line intersecting posts 160 is in perpendicular relation to curved central axis "Cl" (FIG. 2A) of anvil plate 140. With brief reference to FIG. 4A, anvil plate 140 further includes a pair of biasing members 162 each of which being engaged to a respective post of the pair of posts 160. Biasing members 162 may be in the form of coil springs configured to engage cartridge assembly 130 to resiliently bias anvil assembly 112 toward a spaced position from cartridge assembly 130. Biasing members 162 may be bonded to posts 160 via adhesive.

With reference to FIGS. 3A and 3B, anvil cover 180 is shaped and dimensioned for complimentary mating engagement with anvil plate 140. Similar to anvil plate 140, anvil cover 180 has an arcuate configuration defining a curved central axis "C2" that extends between a first end 182a and a second end 182b of anvil cover 180. First end 182a of anvil cover 180 has a pair of flanges 184 extending perpendicularly from lateral sides of anvil cover 180. Flanges 184 each define a proximal opening 185 therethrough configured for receipt of a fastener (not explicitly shown) that facilitates coupling anvil cover 180 with a distal end of proximal elongate portion 102. As such, in its assembled state, anvil assembly 112 is coupled to proximal elongate portion 102 via anvil cover 180.

First end 182a of anvil cover 180 has a finger or tab 186 extending proximally therefrom. Tab 186 has a pair of cutouts 187 formed therein. Cutouts 187 are positioned on each lateral side of tab 186 to help secure anvil cover 180 to proximal elongate portion 102. More particularly, proximal elongate portion 102 includes a channel (not shown) therein, and the channel includes a pair of protrusions (not shown). Tab 186 of anvil cover 180 mechanically engages the channel of proximal elongate portion 102, such that cutouts 187 are aligned with the protrusions. An outer sleeve (not explicitly shown) covers the tab 186 and the channel. The configuration of tab 186 and the channel facilitates a secure connection between anvil cover 186 and proximal elongate portion 102. Moreover, this connection results in a non-movable (e.g., non-pivotable) anvil assembly 112 with respect to proximal elongate portion 102. In some embodiments, anvil assembly 112 may be pivotably coupled to proximal elongate portion 102.

Second end 182b of anvil cover 180 has an opening 188 defined through a thickness thereof. Opening 188 is in alignment with curved central axis "C2" such that upon assembly of anvil plate 140 with anvil cover 180, respective openings 146, 188 of anvil plate 140 and anvil cover 180 are in overlapping alignment.

Anvil cover 180 includes elongate surface features 190 fixed on a surface 192 of anvil cover 180, and disposed along a periphery "P" of anvil cover 180. Elongate surface features 190 of anvil cover 180, and beads 158 of anvil plate 140 are configured for abutting engagement with one another upon assembly of anvil cover 180 with anvil plate 140, as described in greater detail below. Elongate surface features 190 may be monolithically formed with one another to define one unitary elongate surface feature, or may be spaced from one another along periphery "P" as illustrated in FIG. 3A. Elongate surface features 190 have a triangular transverse cross section configuration corresponding to triangular-shaped beads 158 of anvil plate 140. In some embodiments, elongate surface features 190 may assume a variety of shapes and transverse cross section configurations corresponding to respective shapes of the beads 158, such as, for example, square, round, oval, pyramidal, star-shaped, uniform, non-uniform, or the like. It is contemplated that elongate surface features 190 may be fabricated from a variety of materials, such as, for example, those materials already described herein.

With reference to FIGS. 2A-4B, a method of manufacturing/assembling anvil assembly 112 will be described. Anvil plate 140 and anvil cover 180 are placed in complimentary relation to one another by using walls 156 of anvil plate 140 as a guide and by manipulating anvil plate 140 and anvil cover 180 relative to one another until opening 146 defined in second end 142b of anvil plate 140 is aligned with opening 188 defined in second end 182b of anvil cover 180. Anvil plate 140 and anvil cover 180 are further manipulated until beads 158 of first surface 154a of anvil plate 140 are in abutting engagement with longitudinal bead features 190 of surface 192 of anvil cover 180.

Upon properly positioning anvil cover 180 with anvil plate 140, an ultrasonic energy applicator (not shown) directs ultrasonic energy to an interface "I" (FIG. 4B) defined between beads 158 of anvil plate 140 and elongate features 190 of anvil cover 180. The ultrasonic energy melts the interface "I" to ultrasonically weld the the anvil cover 180 to the anvil plate 140, as shown in FIGS. 4A and 4B . This process eliminates the need for sheet metal pressing, laser welding, and other complex machining operations typically used to manufacture the anvil assembly 112. Other forms of welding can be used.

Anvil cover 180, with anvil plate 140 welded thereto, may then be coupled to the remainder of end effector 100.

It will be understood that various modifications may be made to the embodiments of the presently disclosed surgical stapling instruments. Therefore, the above description should not be construed as limiting, but merely as exemplifications of embodiments.

## Claims

1. A pre-assembly for producing an anvil assembly (112) for use with a surgical stapling instrument (10) comprising:
an anvil plate (140) fabricated from a thermoplastic material and having a knife channel (144) defined longitudinally therethrough, the anvil plate including:
a first surface (154a) having a plurality of beads (158) disposed along a periphery thereof; and
a second surface (154b) opposite the first surface; and
an anvil cover (180) fabricated from a thermoplastic material and configured to be coupled to a distal end of the surgical stapling instrument, the anvil cover including a surface (192) having at least one elongate surface feature (190) disposed along a periphery thereof, wherein the plurality of beads (158) of the anvil plate and the at least one elongate surface feature of the anvil cover are configured for abutting engagement upon assembly thereof.

2. The pre-assembly for producing an anvil assembly according to claim 1, wherein at least one bead (158) of the plurality of beads has a triangular configuration; and/or wherein the at least one elongate surface feature (190) has a triangular transverse cross section configuration.

3. The pre-assembly for producing an anvil assembly according to claim 1 or claim 2, wherein the thermoplastic material is polyetheretherketone.

4. The pre-assembly for producing an anvil assembly according to any preceding claim, wherein an end (142b) of the anvil plate and an end (182b) of the anvil cover (180) each define an opening (146, 188) therethrough such that upon assembly of the anvil plate with the anvil cover, the openings are in overlapping alignment.

5. The pre-assembly for producing an anvil assembly according to claim 4, wherein the anvil plate further includes a pair of posts (160) extending perpendicularly from an end of the second surface thereof.

6. A pre-assembly for producing an anvil assembly according to claim 5,
wherein the anvil plate further includes a pair of biasing members (162) engaged to the pair of posts.

7. The pre-assembly for producing an anvil assembly according to any preceding claim, wherein the anvil plate (140) further includes a wall (156) extending perpendicularly from the periphery of the first surface (154a) of the anvil plate.

8. The pre-assembly for an anvil assembly according to any preceding claim, wherein the anvil plate (140) has an arcuate configuration.

9. An end effector for use with a surgical stapling instrument, the end effector including an anvil assembly formed from the pre-assembly according to any one of the preceding claims, further comprising: a cartridge assembly (130), wherein at least one of the anvil assembly (112) or the cartridge assembly is movable relative to the other between a spaced position and an approximated position to engage and staple tissue therebetween.

10. A method of manufacturing an anvil assembly (112) of a surgical stapling instrument (10), the method comprising:
providing an anvil plate (140) fabricated from a thermoplastic material and having a knife channel (144) defined longitudinally therethrough, the anvil plate including:
a first surface (154a) having a plurality of beads (158) disposed along a periphery thereof; and
a second surface (154b) opposite the first surface;
providing an anvil cover (180) fabricated from a thermoplastic material and
configured to be coupled to a distal end of a surgical stapling instrument, the anvil cover including a surface (192) having at least one elongate surface feature (190) disposed along a periphery thereof; and
ultrasonically welding the anvil plate (140) to the anvil cover (180) at an interface between the plurality of beads (158) of the anvil plate and the at least one elongate surface feature (190) of the anvil cover.

11. The method according to claim 10, further comprising aligning an opening (146) defined in an end (142b) of the anvil plate with an opening (188) defined in an end (182b) of the anvil cover prior to ultrasonically welding the anvil plate to the anvil cover.

## Patentansprüche

1. Vormontage zum Erzeugen einer Ambossanordnung (112) zur Verwendung mit einem chirurgischen Klammerinstrument (10), umfassend:
eine Ambossplatte (140), die aus einem thermoplastischen Material gefertigt ist und einen Messerkanal (144) aufweist, der in Längsrichtung dort hindurch definiert ist, wobei die Ambossplatte aufweist:
eine erste Oberfläche (154a), die eine Vielzahl von Perlen (158) aufweist, die entlang eines Umfangs davon angeordnet sind; und
eine zweite Oberfläche (154b), die der ersten Oberfläche gegenüber liegt; und
eine Ambossabdeckung (180), die aus einem thermoplastischen Material gefertigt ist und konfiguriert ist, um mit einem distalen Ende des chirurgischen Klammerinstruments gekoppelt zu werden, wobei die Ambossabdeckung eine Oberfläche (192) aufweist, die zumindest ein längliches Oberflächenmerkmal (190) aufweist, das entlang eines Umfangs davon angeordnet ist, wobei die Vielzahl von Perlen (158) der Ambossplatte und das zumindest eine längliche Oberflächenmerkmal der Ambossabdeckung zum Anlageeingriff bei einer Montage davon konfiguriert sind.

2. Vormontage zum Erzeugen einer Ambossanordnung nach Anspruch 1, wobei zumindest eine Perle (158) der Vielzahl von Perlen dreieckige Konfiguration aufweist; und/oder
wobei das zumindest eine längliche Oberflächenmerkmal (190) eine dreieckige transversale Querschnittskonfiguration aufweist.

3. Vormontage zum Erzeugen einer Ambossanordnung nach Anspruch 1 oder Anspruch 2, wobei das thermoplastische Material Polyetheretherketon ist.

4. Vormontage zum Erzeugen einer Ambossanordnung nach einem der vorstehenden Ansprüche, wobei ein Ende (142b) der Ambossplatte und ein Ende (182b) der Ambossabdeckung (180) jeweils eine Öffnung (146, 188) dadurch definieren, sodass bei einer Montage der Ambossplatte mit der Ambossabdeckung die Öffnungen in überlappender Ausrichtung sind.

5. Vormontage zum Erzeugen einer Ambossanordnung nach Anspruch 4, wobei die Ambossplatte weiter ein Paar von Pfosten (160) aufweist, die sich senkrecht von einem Ende der zweiten Oberfläche davon erstrecken.

6. Vormontage zum Erzeugen einer Ambossanordnung nach Anspruch 5, wobei die Ambossplatte weiter ein Paar von Vorspannelementen (162) aufweist, die mit dem Paar von Pfosten im Eingriff stehen.

7. Vormontage zum Erzeugen einer Ambossanordnung nach einem der vorstehenden Ansprüche, wobei die Ambossplatte (140) weiter eine Wand (156) aufweist, die sich senkrecht von dem Umfang der ersten Oberfläche (154a) der Ambossplatte erstreckt.

8. Vormontage zum Erzeugen einer Ambossanordnung nach einem der vorstehenden Ansprüche, wobei die Ambossplatte (140) eine gekrümmte Konfiguration aufweist.

9. Endeffektor zur Verwendung mit einem chirurgischen Klammerinstrument, wobei der Endeffektor eine Ambossanordnung aufweist, die von der Vormontage nach einem der vorstehenden Ansprüche gebildet wird, weiter umfassend:
eine Kartuschenanordnung (130), wobei zumindest eine von der Ambossanordnung (112) oder der Kartuschenanordnung relativ zu der anderen zwischen einer beabstandeten Position und einer angenäherten Position bewegbar ist, um dazwischen Gewebe in Eingriff zu bringen und zu klammern.

10. Verfahren zum Herstellen einer Ambossanordnung (112) eines chirurgischen Klammerinstruments (10), wobei das Verfahren umfasst:
Bereitstellen einer Ambossplatte (140), die aus einem thermoplastischen Material gefertigt ist und einen Messerkanal (144) aufweist, der in Längsrichtung dort hindurch definiert ist, wobei die Ambossplatte aufweist:
eine erste Oberfläche (154a), die eine Vielzahl von Perlen (158) aufweist, die entlang eines Umfangs davon angeordnet sind; und
eine zweite Oberfläche (154b), die der ersten Oberfläche gegenüber liegt;
Bereitstellen einer Ambossabdeckung (180), die aus einem thermoplastischen Material gefertigt ist und konfiguriert ist, um mit einem distalen Ende des chirurgischen Klammerinstruments gekoppelt zu werden, wobei die Ambossabdeckung eine Oberfläche (192) aufweist, die zumindest ein längliches Oberflächenmerkmal (190) aufweist, das entlang eines Umfangs davon angeordnet ist; und
Ultraschallschweißen der Ambossplatte (140) an die Ambossabdeckung (180) an einer Grenzfläche zwischen der Vielzahl von Perlen (158) der Ambossplatte und dem zumindest einen länglichen Oberflächenmerkmal (190) der Ambossabdeckung.

11. Verfahren nach Anspruch 10, weiter umfassend Ausrichten einer Öffnung (146), die in einem Ende (142b) der Ambossplatte definiert ist, mit einer Öffnung (188), die in einem Ende (182b) der Ambossabdeckung definiert ist, bevor die Ambossplatte mit der Ambossabdeckung mittels Ultraschall verschweißt wird.

## Revendications

1. Pré-ensemble de production d'un ensemble formant enclume (112) destiné à être utilisé avec une agrafeuse chirurgicale (10) comprenant :
une plaque d'enclume (140) fabriquée dans une matière thermoplastique et ayant un canal pour couteau (144) défini longitudinalement à travers, la plaque d'enclume comprenant :
une première surface (154a) ayant une pluralité de bourrelets (158) disposés le long d'une périphérie de celle-ci ; et
une seconde surface (154b) opposée à la première surface ; et
un couvercle d'enclume (180) fabriqué dans une matière thermoplastique et configuré pour être accouplé à une extrémité distale de l'agrafeuse chirurgicale, le couvercle d'enclume comprenant une surface (192) ayant au moins une caractéristique de surface allongée (190) disposée le long d'une périphérie de celle-ci, dans lequel la pluralité de bourrelets (158) de la plaque d'enclume et l'au moins une caractéristique de surface allongée du couvercle d'enclume sont configurés pour venir en prise par butée lors de leur assemblage.

2. Pré-ensemble de production d'un ensemble formant enclume selon la revendication 1, dans lequel au moins un bourrelet (158) de la pluralité de bourrelets présente une configuration triangulaire et/ou dans lequel l'au moins une caractéristique de surface allongée (190) présente une configuration triangulaire en coupe transversale.

3. Pré-ensemble de production d'un ensemble formant enclume selon la revendication 1 ou la revendication 2, dans lequel la matière thermoplastique est le polyétheréthercétone.

4. Pré-ensemble de production d'un ensemble formant enclume selon l'une quelconque des revendications précédentes, dans lequel une extrémité (142b) de la plaque d'enclume et une extrémité (182b) du couvercle d'enclume (180) définissent chacune une ouverture (146, 188) à travers ceux-ci, de telle sorte que, lors de l'assemblage de la plaque d'enclume avec le couvercle d'enclume, les ouvertures se trouvent en alignement par chevauchement.

5. Pré-ensemble de production d'un ensemble formant enclume selon la revendication 4, dans lequel la plaque d'enclume comprend en outre une paire de montants (160) s'étendant perpendiculairement à partir d'une extrémité de la seconde surface de celle-ci.

6. Pré-ensemble de production d'un ensemble formant enclume selon la revendication 5, dans lequel la plaque d'enclume comprend en outre une paire d'éléments de sollicitation (162) mis en prise avec la paire de montants.

7. Pré-ensemble de production d'un ensemble formant enclume selon l'une quelconque des revendications précédentes, dans lequel la plaque d'enclume (140) comprend en outre une paroi (156) s'étendant perpendiculairement par rapport à la périphérie de la première surface (154a) de la plaque d'enclume.

8. Pré-ensemble pour un ensemble formant enclume selon l'une quelconque des revendications précédentes, dans lequel la plaque d'enclume (140) présente une configuration incurvée.

9. Effecteur d'extrémité destiné à être utilisé avec une agrafeuse chirurgicale, l'effecteur d'extrémité comprenant un ensemble formant enclume formé à partir du pré-ensemble selon l'une quelconque des revendications précédentes, comprenant en outre : un ensemble cartouche (130), dans lequel au moins un de l'ensemble formant enclume (112) ou de l'ensemble cartouche est déplaçable par rapport à l'autre entre une position espacée et une position approchée pour mettre en prise et agrafer le tissu entre eux.

10. Procédé de fabrication d'un ensemble formant enclume (112) d'une agrafeuse chirurgicale (10), le procédé comprenant :
la fourniture d'une plaque d'enclume (140) fabriquée dans une matière thermoplastique et ayant un canal pour couteau (144) défini longitudinalement à travers, la plaque d'enclume comprenant :
une première surface (154a) ayant une pluralité de bourrelets (158) disposés le long d'une périphérie de celle-ci ; et
une seconde surface (154b) opposée à la première surface ;
la fourniture d'un couvercle d'enclume (180) fabriqué dans une matière thermoplastique et configuré pour être accouplé à une extrémité distale d'une agrafeuse chirurgicale, le couvercle d'enclume comprenant une surface (192) ayant au moins une caractéristique de surface allongée (190) disposée le long d'une périphérie de celle-ci ; et
le soudage aux ultrasons de la plaque d'enclume (140) au couvercle d'enclume (180) à une interface entre la pluralité de bourrelets (158) de la plaque d'enclume et l'au moins une caractéristique de surface allongée (190) du couvercle d'enclume.

11. Procédé selon la revendication 10, comprenant en outre l'alignement d'une ouverture (146) définie dans une extrémité (142b) de la plaque d'enclume avec une ouverture (188) définie dans une extrémité (182b) du couvercle d'enclume avant le soudage aux ultrasons de la plaque d'enclume au couvercle d'enclume.
